# FASCICULE DE BREVET EUROPEEN

(11) **EP 0 427 843 B1**
(45) Date de publication et mention de la délivrance du brevet: **08.03.1995**
(21) Numéro de dépôt: 90908553.2
(22) Date de dépôt: 01.06.1990
(51) Int. Cl.: A61K 31/28, A61K 31/70

(54) **PRODUIT INHIBITEUR SPECIFIQUE DE LA TRANSCRIPTASE INVERSE DE RETROVIRUS, ET SON APPLICATION COMME MEDICAMENT**
SPEZIFISCHER PRODUKTINHIBITOR DER RETROVIRALEN REVERSEN TRANSKRIPTASE UND SEINE VERWENDUNG ALS ARZNEIMITTEL
SPECIFIC INHIBITOR FOR RETROVIRUS REVERSE TRANSCRIPTASE, AND ITS APPLICATION AS A MEDICAMENT

(30) Priorité: 02.06.1989 FR 8907356
(43) Date de publication de la demande: 22.05.1991
(73) Titulaire: BLOUGH, Herbert A., Dr., Berwyn, Pennsylvania 19312 (US)
(72) Inventeur: BLOUGH, Herbert, A., F-75001 Paris (FR); RICHETTI, Miria, F-75006 Paris (FR); BUC, Henri, F-75015 Paris (FR)
(74) Mandataire: Phélip, Bruno
(86) Numéro de dépôt international: FR9000387
(87) Numéro de publication internationale: WO9014825

(56) Documents cités:
- US-A- 4 657 763
- Dialog Information Services, Base de données 157: Aidsline 1980-90, no. d'accès 01291189, H. Blough et al.: "Organic 'gold' compounds are effecxtive against 'HIV' 1 reverse transcriptase", see the abstract & Int. Conf. AIDS, June 4-9 1989, 5, page 559 (abstract no. M.C.P. 106)
- The Merck Index, An Encyclopedia of Chemicals, Drugs, and Biologicals, 10th edition, 1983, Merck & Co., Inc., (Rahway, N.J., US), pages 126-128 see page 126, no. 882; page 127, no. 888
- J. Am. Chem. Soc., volume 103, 1981, American Chemical Society, K. Brown et al.: "197 Au Mössbauer spectroscopic data for antiarthritic drugs and related gold(I) thiol derivatives", pages 4943-4945 see the whole article
- Anticancer Research, volume 7, 1987, E. De Clercq: "Perspectives for the chemotherapy of AIDS", pages 1023-1038 see pages 1029-1030
- Cancer Research, volume 46, no. 1, 1986 S. Haga et al.: "Conventional and immunocolloidal gold electron microscopy of eight simian retroviruses closely related to human T-cell leukemia virus type I", pages 293-299 see the whole article
- J. Clin Pathol, volume 37, 1984 W.W. Fermemans et al.: "Ultrastructural demonstration of retrovirus antigens with immuno-gold staining in prodromal acquired immune deficiency syndrome", pages 1399-1403 see the whole article

## Description

La présente invention concerne un produit inhibiteur spécifique de la transcriptase inverse de rétrovirus, ainsi qu'une composition médicamenteuse le contenant. La transcriptase inverse sera également dénommée reverse transcriptase.

Les glycoprotéines de virus à enveloppe, se propageant dans les eucaryotes, contiennent la séquence d'acides amines:

Asn-Xaa-Thr

où X = tout acide aminé à l'exception de Pro ou Asp. Cette séquence sert de site accepteur pour des N-oligosaccharides dans le transfert d'intermédiaires liés au dolichol (à la chaîne polypeptidique naissante). Ainsi, toute stratégie chimiothérapeutique, qui est orientée vers le blocage de la synthèse et la charge du transfert du ou des intermédiaires polyprénoliques, ou le traitement des oligosaccharides - pour devenir soit un complexe soit un hydride "à teneur élevée en mannnose" - produira une ou des chaînes oligosaccharidiques raccourcies ou de base. Cette modification des glycoconjugués modifiera la structure tertiaire et quaternaire des protéines de fixation des virus et du (ou des) récepteur(s), faisant avorter l'entrée du virus: fixation, fusion (cellule à cellule, virus à cellule), de même que sa libération. Lorsqu'il est utilisé de façon appropriée, le 2-désoxy-D-glucose (2dGlc) possède une activité antivirale unique et il a été utilisé avec succès pour traiter l'herpès génital [Blough H.A. et al.: Herpes virus and virus chemotherapy (R. Kono ed.), pages 211-214, Elsevier, Amsterdam (1985); Blough H.A. et Giuntoli R., J. Am. Med. Assoc. 241, 2798-2801 (1979)].

Plus récemment, il a été démontré que le 2dGlc présente une activité antivirale à l'encontre de HIV et d'autres rétrovirus, ainsi que leurs protéines de fixation [Blough H.A. Pauwels R., de Clercq E., Conieaux J., Goldberger S. & Thiry L., Biochem. Res. Commun. 141, 31-36 (1986); Blough H.A. et al.: Herpes virus and virus chemotherapy (R. Kono ed.), pages 211-214, Elsevier, Amsterdam (1985)].

Les présents inventeurs ont été les premiers à démontrer que des inhibiteurs de glycosylation étaient efficaces pour bloquer la fixation, la pénétration et la fusion de HIV et d'un certain nombre de rétrovirus [Blough H.A., Biochem. Biophys. Res. Commun. 141, 31-36 (1986); Blough H.A. et al: Herpes virus and virus chemotherapy (R. Kono ed.), pages 211-214, Elsevier, Amsterdam (1985); Thiry L. Blough H.A. et al on "Viral Pathophysiology" (R, Gallo, ed.) Italie (1984)], à savoir le virus de la leucémie bovine et le virus endogène du babouin. En fait, il a été montré que les inhibiteurs de glycosylation pouvaient induire la lyse des cellules infectées par les rétrovirus (par exemple, cellules infectées de façon chronique par le virus endogène de babouin). Dans le cas de l'herpès virus [Gallaher W.R. Levitan D.B. & Blough H.A., Virology 55, 193-202 (1973)], les infections chez l'homme ont été traitées avec succès [Thiry L. et al. indiqué ci-dessus; Blough H.A. et Giuntoli R., J. Am. Med. Assoc. 241, 27998-2801 (1979)]. Plus de 7000 patients ont été traités avec succès par des composés tels que le 2-désoxy-D-glucose (cf. brevets américains N° US-A-4315001 et US-A-4603122).

Par ailleurs, deux des médicaments couramment disponibles pour le traitement du SIDA, à savoir la 5'-azidothymidine ou zidovudine (AZT) et la 3',5'-didésoxycytidine se sont avérés toxiques. De plus, ils nécessitent des kinases cellulaires pour les phosphoryler, sinon ils ne sont pas actifs.

Les présents inventeurs ont choisi d'utiliser des composés qui agissent directement sur la transcriptase inverse (clonée et/ou purifiée), et ils ont découverts que l'addition d'or aux analogues du glucose et à certains composés aliphatiques du type alcool, de façon à obtenir, pour ces composés, au moins une liaison S-Au, produisait une action antivirale efficace à l'encontre de la reverse transcriptase de rétrovirus tels que HIV-1 et AMV.

Plusieurs de ces composés sont disponibles pour le traitement de l'arthrite rhumatoïde [Payne B.H. & Walz D.T., Vet. Pathol. 15, suppl. 5,1 (1979); Symposium: J. Rheumatol. 9, 1-209 (1982)] (comme immunosuppresseurs) et sont disponibles en Europe de l'Ouest et aux Etats-Unis pour le traitement de cette dernière maladie.

La présente invention a donc d'abord pour objet l'utilisation d'un thio-composé organique ayant au moins une liaison S-Au pour la fabrication d'un médicament destiné au traitement et à la chimioprophylaxie chez l'homme et chez l'animal des maladies dues aux rétrovirus.

Ce thio-composé est notamment un thio-glucose. En particulier, celui-ci est un glucose ou un analogue du glucose dont un OH est substitué par un groupement SAu ou un groupement SAuR, où R représente un reste de molécule organique. Le thio-glucose peut comporter également une chaîne acyle grasse.

Des exemples de thio-glucose sont le 1-bêta-D-aurothioglucose, de formule: 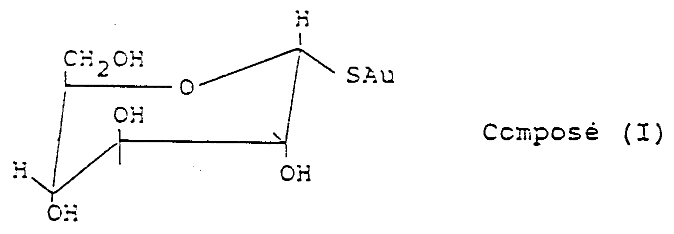
et le 2,3,4,6-tétra-0-acétyl-1-thio-bêta-D-glucopyranasato-5-triacétyl-phosphine-or, de formule 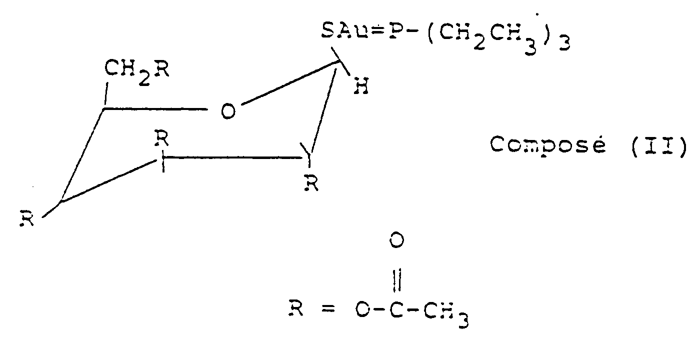
Ce dernier composé est commercialisé sous la marque de fabrique AURANOFIN®.

On peut également envisager, comme thio-composé, des thio-composés aliphatiques, comprenant une fonction -SAu. Un exemple de ce thio-composé à fonction -SAu est le composé de formule

CH₃-CH₂-CH₂-S-Au.Na₂SO₃

La présente invention porte également sur une composition médicamenteuse, caractérisée par le fait qu'elle renferme, à titre de principe actif, en une quantité efficace du point de vue pharmacologique ou vétérinaire, au moins un composé aliphatique, à l'exclusion de l'aurothiomalate et de l'aurothiopropanolsulfonate, tel que défini ci-dessus.

En particulier, cette composition médicamenteuse est destinée au traitement et à la chimioprophylaxie des maladies provoquées par les virus de l'immunodéficience humaine HIV-I, HIV-2, HTLV1, HTLV2, par le virus AMV ( Avian Myoblastosis Virus), par le virus FLV (Felian Leukemia Virus) et par le virus EIAV (Equine Infectious Anemia Virus).

De façon particulièrement avantageuse, dans la composition médicamenteuse de l'invention, le principe actif est piégé au sein de liposomes. On peut avantageusement utiliser à cet effet la technique décrite par Baron C. & Blough H.A., Intervirology 9, 33-39 (1985), avec le détergent neutre octyl glucoside. On peut ainsi préparer des liposomes avec une ou des molécules de CD₄ et des quantités accrues de phosphatidylsérine par rapport à la phosphatidylcholine et au cholestérol. Les composés de l'invention seraient donc piégés à l'intérieur des liposomes à l'état stable. Toutes les préparations devraient être stockées en verrerie actinique et sous atmosphère inerte. Cette technique permet un accès rapide de ces composés au milieu intracellulaire et augmente la perméabilité des cellules du système nerveux central et des monocytes à ces composés. En variante, on peut utiliser des composés modifiés par l'addition d'une chaîne acyle grasse de C₃₋₈, similaire à celle nécessaire à la synthèse d'octylglucoside. Cette technique permet un accès rapide de ces composés au milieu intracellulaire et augmente la perméabilité des cellules du système nerveux central et des monocytes. Ces composés, comme les propyl-, l'hexyl-, l'octyl-auriothioglucosides, peuvent être synthétisés (Baron C. et Blough H.A., 1985, Intervirology, 9, 33, 39).

En eux-mêmes, les composés de l'invention sont stables en solutions aqueuses pendant au moins 2 semaines à - 20°C.

Pour cette composition, le principe actif peut être associé à au moins un inhibiteur de glycosylation, tel que le 2-désoxy-D-glucose, qui bloque l'entrée (fixation, pénétration, fusion) de HIV-I, ou à la zidovudine (AZT) ou un de ses dérivés, notamment un dérivé phosphorylé (zidovudine triphosphate).

En particulier, la composition médicamenteuse selon l'invention se présente sous une forme administrable par voie intraveineuse. Elle peut également être utilisée par voie orale.

Il a été démontré que les composés contenant la fonction SAu présentaient l'activité recherchée. Des produits de base comme le 1-bêta-D-thioglucose et le 5-bêta-D-thioglucose étaient actifs à l'encontre de la glycosylation de gp120 ou gp41, cependant à des concentrations dépassant 2mM, ils étaient cytotoxiques, mais ne présentaient aucun effet sur la polymérase.

Il a également été montré que les composés de l'invention sont moins toxiques que leurs analogues de base, en mesurant, à l'aide du microscope photonique, le temps de duplication cellulaire et l'exclusion du bleu trypan.

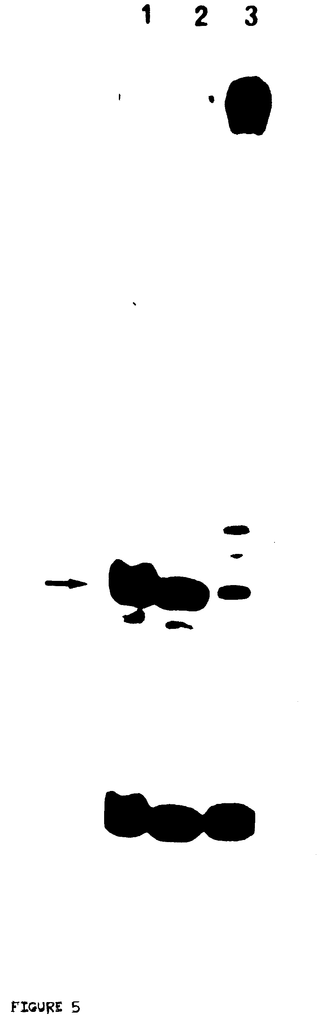

L'invention sera encore illustrée sans être aucunement limitée par les exemples qui suivent, et qui sont illustrés par les Figures 1 à 8.

Les Figures 1 et 2 sont des autoradiogrammes représentant l'action de l'aurothioglucose sur l'activité des transcriptases inverses HIV1-RT et AMV-RT.

La Figure 3 est un autoradiogramme comparant l'action de l'aurothioglucose sur la transcriptase inverse en utilisant des matrices ARN et ADN. Les concentrations de 1-aurothioglucose sont comprises entre 1 et 100 micromoles.

La Figure 4 est un autoradiogramme représentant l'effet de la concentration en désoxyribonucléotide (TP3, puits 1 à 4) et de concentration en transcriptase inverse (puits 5 à 20) sur l'activité in vitro de l'aurothioglucose.

La Figure 5 représente un autoradiogramme montrant les effets sur l'élongation des chaînes d'ADN par l'AMV-RT de l'aurothioglucose. Le puits 1 correspond à l'arrêt de la réaction de polymérisation de nucléotides par l'AMV-RT, en présence de dATP, TTP, sans addition d'inhibiteurs. La flèche représente la bande correspondant aux six nucléotides.

Le puits 2 représente l'inhibition de l'élongation de la chaîne par addition de l'auro-thioglucose après polymérisation de six nucléotides par l'AMV-RT, en présence de d-ATP, de TTP et de dNTP. Le puits 3 représente l'élongation de la chaîne, la réaction n'étant pas stoppée, sans inhibiteur, après polymérisation de six nucléotides par AMV-RT.

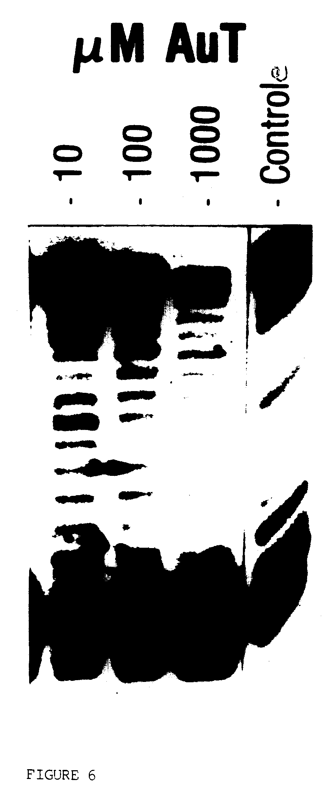

La Figure 6 représente l'inhibition de la DNA-polymérase-1 d'Escherichia Coli par l'aurothioglucose.

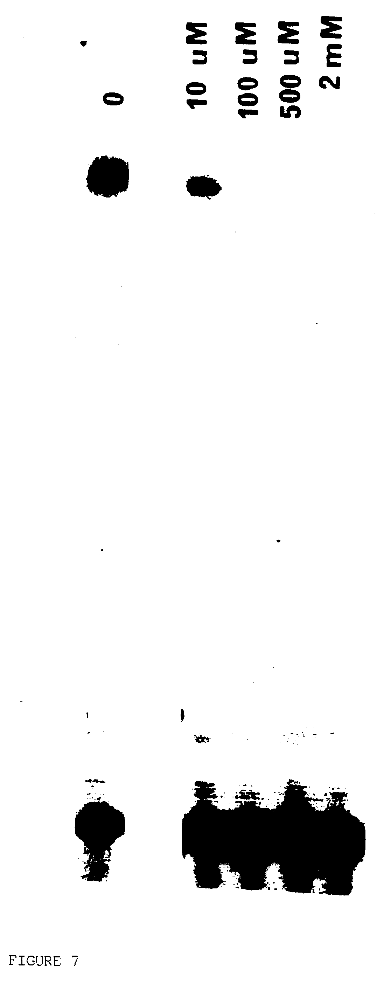

La Figure 7 représente l'inhibition de la transcriptase inverse HIV1-RT par l'aurothiopropanol.

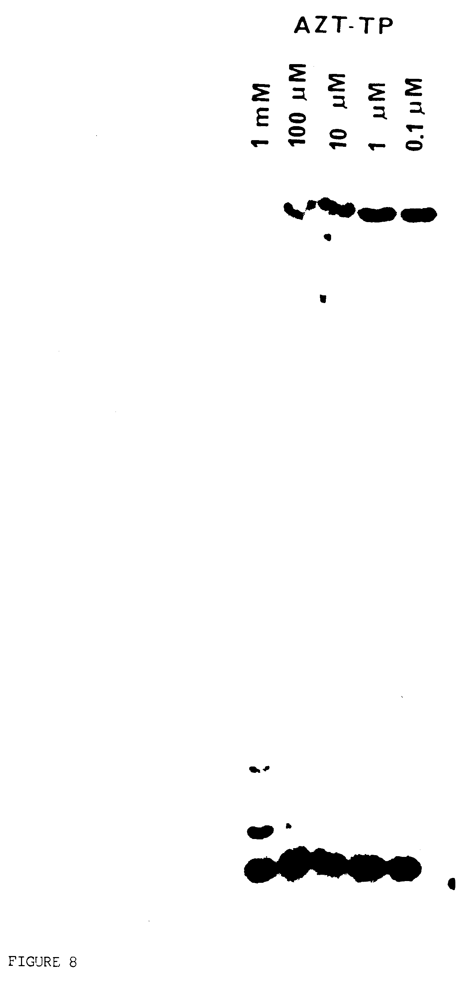

La Figure 8 représente l'inhibition de la HIV1-RT par l'AZT phosphorylé.

### MODES OPERATOIRES

La réplication virale a été testée par prétraitement des cellules infectées (infection chronique) et/ou prétraitement de cellules CEM ou MT-4 non-infectées avant l'infection par HIV-1. Le virus partiellement purifié a été testé pour la réplication virale par la mesure de l'activité R.T. (Reverse Transcriptase); en outre, ces préparations virales lysées ont été incubées avec diverses concentrations d'inhibiteurs potentiels, comme décrit par Blough H.A. et al, Biochem. Biophys. Commun. 141, 31-36 (1986).

Effets de médicaments sur la synthèse de glycoprotéines: des cellules CEM ou MT-4 ont été infectées par HIV-1; 4 heures après l'infection, une moitié des cellules a été marquées avec [2³H]Man ou [6³H]GlcNAc à 2,5-5,9 »Ci. Les milieux ont été changés et de l'inhibiteur frais (potentiel) et 2,5 »Ci (ou 5 »Ci) de l'isotope approprié ont été ajoutés. Les cellules ont été lysées à trois et sept jours après l'infection et les lysats cellulaires ont été clarifiées par centrifugation à 12 000 g; qp120 et gp41 ont été l'une et l'autre isolées par immunoprécipitation sur colonnes et les produits, analysés sur gels de polyacrylamide à 12% et fluorographie [Kumarasamy R & Blough H.A., Biochem. Biophys. Res. Commun. 109, 1108-1115 (1982)]. Les virus ont été récoltés et purifiés par centrifugation à vitesse contrôlée sur gradients préformés de citrate de sodium ou métrazamide à 5-40%, et analysés de façon similaire.

Fusion: des cellules infectées de façon chronique ou des cellules MT-4 non infectées ont été prétraitées par divers inhibiteurs et la fusion a été testée (nombre de polycaryocytes) comme décrit par Blough H.A. et al., Biochem. Biophys. Res. Commun. 141, 31-36 (1986).

Transcriptase inverse: l'enzyme active du point de vue enzymatique, purifiée, clonée, a été préparée dans Escherichia coli à l'aide de la technique précédemment décrite par Hizi A., McGill C., Hugues G., Proc. Natl. Acad. Sci. USA 85, 1218-1222 (1988). Le dosage de la R.T. a été effectuée à l'aide de matrice d'ADN (au hasard et avec des séquences HIV) et de primer(s) marqué(s) (brins d'initiation) par radioactivité. Après formation de complexes entre la matrice d'ADN et les primers, la R.T. a été ajoutée et incubée. Diverses concentrations d'inhibiteur ont été ajoutées soit avant soie après que le, ou les complexes, aient été formés. L'initiation et l'élongation de la chaîne ont été étudiées à l'aide de P.A.G.E. à 20% [Shane D.L., Goodman M.F., Echols N.A., Nucleic Acids Res. 16, 1218-1222 (1988] et les produits analysés par autoradiographie. Différentes matrices et conditions ont été utilisées pour distinguer ces fonctions. En outre, de la R.T. disponible dans le commerce provenant du virus de myéloblastose aviaire a été également utilisée.

Le dosage de l'effet des inhibiteurs sur la R.T. était le suivant:
A) On prépare un mélange réactionnel de 17 l contenant:
   . ADN matrice 10⁻⁸M
   . ADN primer 4 x 10⁻¹⁰M/ADN primer 5' [³²P]
   . 2 »l de tampon HIV-RT ([10x] (500 mM Tris-HCl
   . pH 7,8; 60 mM MgCl₂; 10 mM dithiothréitol; 500 mM KCl); et
   . de l'eau bidistillée pendant 20 nm à 37°C.
B) On ajoute 1 unité de HIV-RT (une unité correspond à l'activité enzymatique qui incorpore 1 nmol de [³H] TMP en produit insoluble dans l'acide en 10 mn à 37°C avec du poly(A)-(dT)₁₅ comme substrat) pendant 10 min à 37°C.
C) On ajoute un mélange de désoxy nucléotide triphosphates (dXTPs) contenant 0,25 mM de chaque dXTP pour un volume de mélange final de 20 »l pendant 5 minutes à 37°C.
D) Pour tester le ou les inhibiteurs, diverses concentrations de ces produits (sous un volume constant) sont ajoutées (selon Etape B) au mélange des dXTPs. Le mélange réactionnel est incubé à 37°C pendant 10 nM.
E) On fait cesser la réaction par de l'EDTA 10 nM dans le formamide à 90%.
F) Des échantillons sont chauffés à 90°C pendant 5 mn et les produits séparés par électrophorèse sur gels de Polyacrylamide à 20% en présence d'urée.
G) Les variations dans l'élongation, la synthèse, etc... sont détectées par autoradiographie et testées par comparaison avec des "témoins" sans inhibiteur.

Les tests effectués sur la HIV-RT et l'AMV-RT, avec le 1-aurothioglucose comme inhibiteur ont fourni des autoradiogrammes selon les figures respectivement 1 et 2.

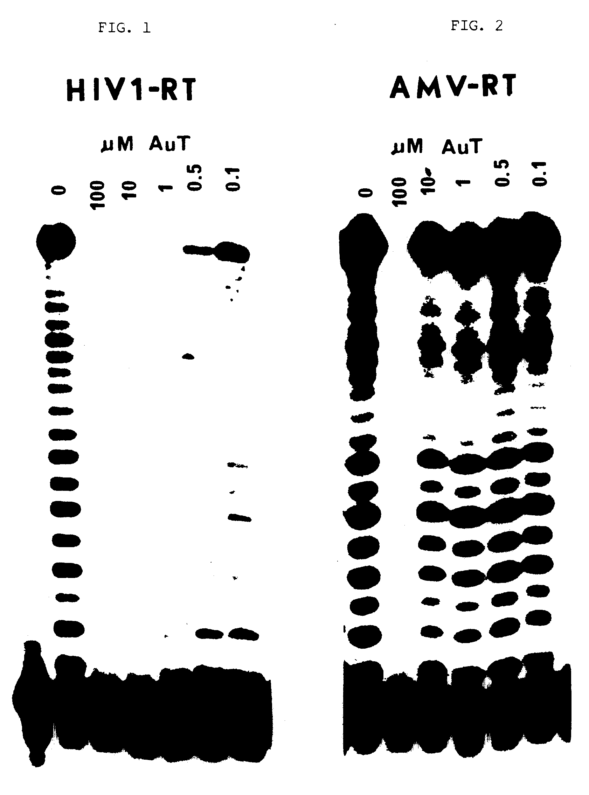

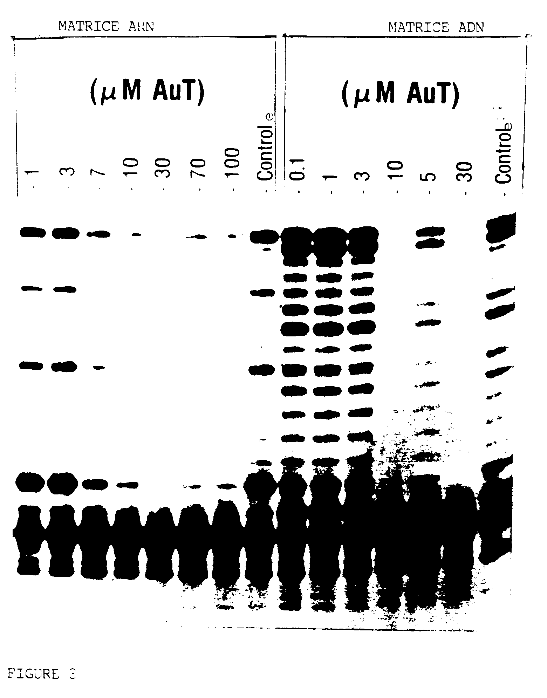

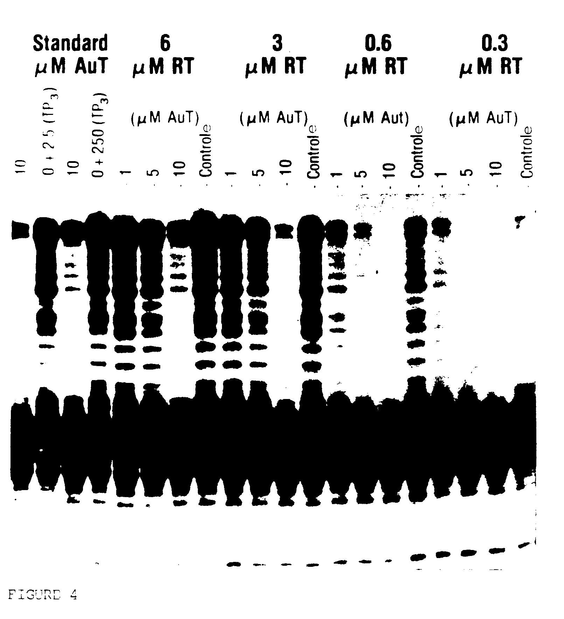

Une quantité aussi faible que 3 »m de 1-aurothioglucose s'est révélée efficace pour bloquer le complexe HIV-R.T.-Substrat à 40 nm (Figure 1). Des résultats comparables ont été obtenus en utilisant des matrices d'ARN, comme le montre la Figure 3 qui représente un autoradiogramme obtenu avec diverses concentrations de matrices ADN et ARN. De même, cet inhibiteur peut agir directement sur l'enzyme libre, à savoir par incubation avec les analogues substitués par Au avec de l'enzyme non complexée. Des résultats comparables ont été obtenus avec AMV (Figure 2), excepté que des concentrations supérieures en 1-aurothioglucose étaient nécessaires.

Des tests ont aussi été effectués afin de déterminer l'influence de la concentration des désosyribonucléotides (dNTP)sur l'action inhibitrice du 1-aurothioglucose.

A cet effet on a effectué des expérimentations en utilisant le procédé précédemment décrit, avec des matrices ADN et ARN, en faisant varier la concentration des NTP de 250 nM à 250 »m.

Aucune variation de la constante d'inhibition (Ki) de l'aurothioglucose n'est observée, ce qui suggère que cette molécule à un site de liaison différent des dNTP, comme le montre l'autoradiogramme des puits 1 à 4 de la Figure 3.

On a aussi testé l'influence de la concentration en enzyme HIV1-RT sur l'activité inhibitrice de l'aurothioglucose (puits 5 à 20 de la Figure 4).

### - Effet du 1-aurothioglucose à différents stades de la polymérisation

De nombreux arguments indiquent que certaines polymérases sont plus résistantes aux effets inhibiteurs de diverses drogues quand elles sont liées à leurs matrices ou activement engagées dans la rétro-transcription (polymérisation du DNA).

Il a donc été testé pour le 1-aurothioglucose l'efficacité à l'encontre de la transcriptase inverse du virus HIV1 (HIV1-RT) au cours de l'étape d'élongation par comparaison à l'efficacité vis-à-vis de l'enzyme libre en solution.

L'inhibition par le 1-aurothioglucose a donc été testée:
a) sur l'enzyme libre
b) sur l'enzyme complexé à la matrice,
c) sur l'enzyme au cours d'une phase d'élongation.

Les tests sont mis en oeuvre comme décrits précédemment:
- dans le cas (a) l'enzyme est préincubé avec diverses concentrations de 1-aurothioglucose pendant 5 mn à 30°C dans le tampon de réaction, puis l'hybride primer (brin d'initiation) - matrice et les désoxy-ribonucléotides (dXTP) sont ajoutés au mélange qui est incubé pendant 5 mn à 37°C.
- dans le cas (b) l'enzyme est incubée avec l'hybride primer-matrice pendant 10 mn à 37°C, puis le 1-aurothioglucose à différentes concentrations et les désoxyribonucloétides (dXTP) sont ajoutés. Le mélange est incubé pendant 5 mn à 37°C.
- dans le cas (c) l'enzyme, l'hybride primer-matrice et 2 des 4 désoxy-ribonucléotides sont ajoutés et incubés pendant 5 mn à 37°C. Les deux autres désoxyribonucléotides (dTTP) sont ajoutés ensemble avec des concentrations augmentées de 1-aurothioglucose et incubées pendant 5 mn à 37°C. Dans ce cas la HIV1-RT a effectué plusieurs cycles d'élongations avant l'addition de la 1-aurothioglucose.

Dans les cas (b) et (c) il a été observé la même sensibilité à l'encontre de la 1-aurothioglucose. Par contre dans le cas de l'expérimentation avec de l'enzyme libre on remarque une légère augmentation de la sensibilité à l'inhibition par la 1-aurothioglucose, la constante d'inhibition (Ki) diminuant de 33%.

### - Effet du 1-aurothioglucose sur l'association enzyme-matrice

Une expérience a été mise en oeuvre selon le protocole décrit dans le cas (a), et on a mesuré la variation de la constante d'inhibition (Ki) en fonction de la concentration en enzymes. On a montré que la constante (Ki) augmente quand la concentration en enzyme est augmentée.

On aussi effectué la filtration de l'enzyme et de l'hybride primeur-matrice en présence de concentration croissante de l'aurothioglucose. Les conditions expérimentales sont celles décrites précédemment.

Il a été montré que l'association enzyme-matrice a été affaiblie dans ces conditions d'expérimentation (la concentration enzymatique varie de 0,15 nM à 1nM, et la concentration en aurothioglucose varie de 0,1 »M à 100 »M.

L'action inhibitrice de l'aurothioglucose s'exerce donc sur l'enzyme et est indépendante de la séquence de la matrice considérée (ADN ou ARN). Elle ralentit la fixation de l'enzyme à son site sur la matrice.

De plus l'aurothioglucose a une activité inhibitrice faible sur les DNA, polymèrases humaines, in vitro.

Composés de base: par exemple, le 1-thioglucose, le 5-thioglucose se sont révélés efficaces sur la glycosylation à des concentration d'environ 3,5 nM (in vitro sur cellules ALEX et MT-4), mais n'ont pas présenté d'effet sur la R.T. de l'un ou l'autre virus. Ils étaient trop toxiques vis-à-vis des cellules pour que l'on mette en évidence leur efficacité clinique.

Un effet inhibiteur a par contre été observé pour l'aurothiopropanol, comme le montre la Figure 7 qui présente un autoradiogramme de préparations contenant de la HIV1-RT et des concentrations de 0 à 2 mM d'aurothiopropanol.

Des études montrent que le mode d'action du composé (I) est totalement différent de celui de l'AZT triphosphate et qu'il est considérablement plus actif que ce dernier.

Ces études sont illustrées sur la Figure 8 qui représente un autoradiogramme de préparations contenant de la transcriptase inverse de HIV1 (HIV-1-RT) et des concentrations de 0,1 »M à 1 mM en AZT. Ces résultats comparés aux résultats de la Figure 1 montrent que le 1-aurothioglucose est 1500 fois plus actif que le 3'-Azido-DTTP (AZT).

On a d'autre part montré que les effets de la zidovudine (AZT) et de l'aurothioglucose sont additifs et qu'il existe une action synergique de ces deux composés.

On a ainsi montré que l'association zidovudine (AZT)/aurothioglucose permet de réduire la dose d'AZT nécessaire pour observer une demiinhibition de la transcriptase inverse.

Les nouveaux composés de l'invention sont administrables par voie intraveineuse ou intramusculaire. Les compositions correspondantes peuvent être présentées sous forme de soluté injectable par exemple avec un composé à 35% d'or dans un véhicule aqueux pour injection. Le soluté peut être mis dans une ampoule avec une solution de chlorure de sodium à 0,9% pour injection à pH très voisin de 7, par exemple dans une ampour le 2ml.

La posologie dépend de la maladie à traiter et de la gravité de l'affection. Au début du traitement, la fréquence d'administration peut être de quatre à six doses réparties en une ou deux fois par semaine. Ensuite on peut pratiquer deux injections par mois pendant trois mois, puis une injection par mois. Dans le cas du traitement du SIDA, on peut prévoir une administration une fois par mois pendant six mois, en vue de la prophylaxie, pour changer la séropositivité, le traitement peut être pratiqué tous les trois mois et répété, par exemple deux fois.

Chez l'homme la quantité actif par dose administrée peut varier entre 25 et 100 mg environ, soit de 1,5 et 2,5 mg par kilo de poids corporel.

En vue de l'administration orale on prévoit les véhicules conventionnels appropriés, par exemple sous forme de capsules ou de dragées avec des stabilisants et des additifs appropriés, tels que le lactose. La quantité de produit actif par dose peut varier chez l'homme entre 2,5 et 5,0 mg par kilo de poids corporel. L'administration peut être quotidienne pendant les premiers mois, par exemple les trois premiers mois et ensuite plus espacée, notamment tous les deux jours.

### - Effet du 1 aurothioglucose sur diverses DNA polymérases:

Plusieurs DNA polymérases ont été testées :
- la DNA polymérase alpha de placenta humain (fournie par le Docteur Olga LAVRIK, Novosibirsk URSS).
- la DNA polymérase alpha de foie de rat partiellement purifiée (fournie par le Docteur A M de RECONDO, de Villejuif France).
- la DNA polymérase I de Escherichia coli (fournie par BRL)
Ces expériences montrent que la DNA polymérase alpha humaine est insensible à l'inhibition par l'aurothioglucose, pour des concentrations en drogue inférieure ou égale à 1 mM, tandis la DNA polymérase alpha de rat est sensible à l'aurothioglucose, mais seulement pour des concentrations supérieures à 100 uM, et la polymérase A d'Escherichia coli est sensible à des concentrations en aurothioglucose supérieures à 50 uM.

La figure 6 est un autoradiogramme représentant l'action de l'aurothioglucose sur la DNA-polymérase I d'Escherichia coli.

L'administration des compositions contenant les composés selon l'invention peut être aussi à l'aide de liposomes.

Les liposomes peuvent être constitués par exemple de dimpalimitoyl-phosphatidylcholine : cholestérol : dicetylphosphate dans des rapports 2:1,5:0,22.

Des liposomes contenant l'aurothioglucose peuvent être préparés comme décrit précèdement pour la préparation de liposomes contenant de la ribavirine (KENDE ET ALTER, Antimicrob Agents and Chem Ther, 27 : 903-907, 1985).

De tels liposomes peuvent contenir 25% d'aurothioglucose, dont la quantité est déterminée par absorption atomique.

On peut aussi préparer de tels liposomes par solubilisation dans un détergent et dialyse d'octyl glucoside comme décrit précédement par BARON (Intervirology, 9:33-43,1983).

De tels liposomes contenant de l'aurothioglucose sont administrés par injection intra-péritonale à de femelles de souris Swiss agées de cinq à six semaines, en utilisant l'équivalent de 37,5 microgrammes de thio-auroglucose par 25 grammes de poids corporel de souris. L'injection est répétée tous les jours pendant douze jours. Après une période de quatorze jours on observe aucun signe de toxicité manifeste et aucune léthalité (sur six souris).

Les indications sont données bien entendu à titre général, et doivent être adaptées aux conditions particulières de la maladie en cause. On veuillera aussi aux contre indications : cas de grossesse, d'insuffisance hépatique ou rénale, de traitement des enfants.

## Revendications (Revendications pour l'(les) Etat(s) contractant(s) suivant(s): AT, BE, CH, DE, DK, FR, GB, IT, LI, LU, NL, SE)

1. Utilisation d'un thiocomposé organique ayant au moins une liaison S-Au pour la fabrication d'un médicament destiné au traitement et à la chimioprophylaxie chez l'homme et chez l'animal des maladies dues aux rétrovirus.

2. Utilisation selon la revendication 1, caractérisée en ce que le composé est un thio-glucose.

3. Utilisation selon la revendication 2, caractérisée par le fait que le thio-glucose est un glucose ou un analogue du glucose dont un OH est substitué par un groupement SAu ou un groupement SAuR, où R représente un reste de molécule organique.

4. Utilisation selon l'une des revendications 2 et 3, caractérisée par le fait que le thio-glucose est un thio-glucose modifié par l'addition d'une chaîne acyle grasse en C₃₋₈.

5. Utilisation selon la revendication 4, caractérisée par le fait que le thio-glucose modifié est un propyl-, hexyl-, octyl-aurothioglucoside.

6. Utilisation selon la revendication 3, caractérisée par le fait que le thio-glucose est le 1-bêta-D-aurothioglucose.

7. Utilisation selon la revendication 3, caractérisée par le fait que le thio-glucose et le 2,3,4,6-tétra-O-acétyl-1-thio-bêta-D-glucopyranasato-S-triacétylphosphine-or.

8. Utilisation selon la revendication 1, caractérisée par le fait que le thio-composé est aliphatique.

9. Utilisation selon la revendication 8, caractérisé par le fait que le thio-composé aliphatique à fonction -SAu est le composé de formule :
CH₃-CH₂-CH₂-S-Au Na₂SO₃

10. Utilisation selon l'une des revendication 1 à 9, caractérisé par le fait que le thio-glucose est associé à la zidovudine (AZT) où à un de ses dérivés, notamment un dérivé phosphorylé.

11. Composition médicamenteuse caractérisée par le fait qu'elle renferme, à titre de principe actif , une quantité efficace du point de vue pharmacologique ou vétérinaire d'un thio-composé organique , aliphatique ayant au moins une liaison S-Au,à l'exclusion de l'aurothiomalate et de l'aurothiopropanolsulfonate

12. Composition selon la revendication 11, caractérisée par le fait que le thio-composé aliphatique à fonction -S-Au est le composé de formule:
CH₃-CH₂-CH₂-S-Au.Na₂SO₃

13. Composition médicamenteuse selon l'une des revendications 11 et 12 , caractérisée par le fait qu'elle est destinée au traitement des maladies provoquées par les virus HIV-1, HIV-2, HTLV-1, HTLV-2, AMV, ELAV et FLV .

14. Composition médicamenteuse selon l'une des revendications 11 à 13 , caractérisée par le fait que le principe actif est piégé au sein de liposomes .

15. Composition médicamenteuse selon l'une des revendications 11 à 14 , caractérisée par le fait que le principe actif est associé à au moins un inhibiteur de glycosylation, tel que le 2-désoxy-D-glucose .

16. Composition selon l'une des revendications 11 à 15 caractérisée par le fait que le composé thio organique est associé à la zidovudine (AZT) ou à un de ses dérivés, notamment un dérivé phosphorylé.

17. Composition médicamenteuse selon l'une des revendications 11 à 16 , caractérisée par le fait qu'elle se présente sous une forme administrable par voie intraveineuse .

18. Composition médicamenteuse selon l'une des revendications 11 à 17 , caractérisée par le fait qu'elle se présente sous une forme administrable par voie orale.

## Revendications (Revendications pour l'(les) Etat(s) contractant(s) suivant(s): ES)

1. Utilisation d'un thiocomposé organique ayant au moins une liaison S-Au pour la fabrication d'un médicament destiné au traitement et à la chimioprophylaxie chez l'homme et chez l'animal des maladies dues aux rétrovirus.

2. Utilisation selon la revendication 1, caractérisée en ce que le composé est un thio-glucose.

3. Utilisation selon la revendication 2, caractérisée par le fait que le thio-glucose est un glucose ou un analogue du glucose dont un OH est substitué par un groupement SAu ou un groupement SAuR, où R représente un reste de molécule organique.

4. Utilisation selon l'une des revendications 2 et 3, caractérisée par le fait que le thio-glucose est un thio-glucose modifié par l'addition d'une chaîne acyle grasse en C₃₋₈.

5. Utilisation selon la revendication 4, caractérisée par le fait que le thio-glucose modifié est un propyl-, hexyl-, octyl-aurothioglucoside.

6. Utilisation selon la revendication 3, caractérisée par le fait que le thio-glucose est le 1-bêta-D-aurothioglucose.

7. Utilisation selon la revendication 3, caractérisée par le fait que le thio-glucose et le 2,3,4,6-tétra-O-acétyl-1-thio-bêta-D-glucopyranasato-S-triacétylphosphine-or.

8. Utilisation selon la revendication 1, caractérisée par le fait que le thio-composé est aliphatique.

9. Utilisation selon la revendication 8, caractérisée par le fait que le thio-composé aliphatique à fonction -SAu est le composé de formule:
CH₃-CH₂-CH₂-S-Au Na₂SO₃

10. Utilisation selon l'une des revendications 1 à 9, caractérisée par le fait que le thio-glucose est associé à la zidovudine (AZT) où à un de ses dérivés, notamment un dérivé phosphorylé.

11. Procédé de préparation d'une composition médicamenteuse caractérisé par le fait que l'on associe, à titre de principe actif, une quantité efficace du point de vue pharmacologique ou vétérinaire d'un thio-composé organique, aliphatique ayant au moins une liaison S-Au, à l'exclusion de l'aurothiomalate et de l'aurothiopropanolsulfonate et un véhicule approprié.

12. Procédé selon la revendication 11, caractérisé par le fait que le thio-composé aliphatique à fonction -S-Au est le composé de formule:
CH₃-CH₂-CH₂-S-Au.Na₂SO₃

13. Procédé selon l'une des revendications 11 et 12, caractérisé par le fait que la composition est destinée au traitement des maladies provoquées par les virus HIV-1, HIV-2, HTLV-1, HTLV-2, AMV, ELAV et FLV.

14. Procédé selon l'une des revendications 11 à 13, caractérisé par le fait que le principe actif est piégé au sein de liposomes.

15. Procédé selon l'une des revendications 11 à 14, caractérisé par le fait que le principe actif est associé à au moins un inhibiteur de glycosylation, tel que le 2-désoxy-D-glucose.

16. Procédé selon l'une des revendications 11 à 15, caractérisé par le fait que le composé thio organique est associé à la zidovudine (AZT) ou à un de ses dérivés, notamment un dérivé phosphorylé.

17. Procédé selon l'une des revendications 11 à 16, caractérisé par le fait que la composition se présente sous une forme administrable par voie intraveineuse.

18. Procédé selon l'une des revendications 11 à 17, caractérisé par le fait que la composition se présente sous une forme administrable par voie orale.

## Claims (Claims for the following Contracting State(s): AT, BE, CH, DE, DK, FR, GB, IT, LI, LU, NL, SE)

1. Use of an organic thio-compound having at least an S-Au bond to make a drug useful for the treatment or chimioprophylaxis of diseases caused by retroviruses in the human or animal.

2. Use according to claim 1, characterized in that the compound is a thio-glucose.

3. Use according to claim 2, characterized in that the thio-glucose is a glucose or a glucose analog in which one OH is substituted by an SAu group or an SAuR group in which R represents an organic radical.

4. Use according to one of claims 2 and 3, characterized in that the thio-glucose is a thio-glucose modified by the addition of a C₃-C₈ fatty acyl chain.

5. Use according to claim 4, characterized in that the modified thio-glucose is a propyl-, hexyl-, octyl-aurothio-glucoside.

6. Use according to claim 3, characterized in that the thio-glucose is 1-beta-D-aurothio-glucose.

7. Use according to claim 3, characterized in that the thio-glucose is (2, 3, 4, 6-tetra-o-acetyl-1-thio-beta-D-glucopyranasato-S-triacetylphosphine-gold.

8. Use according to claim 1, characterized in that the thio-compound is aliphatic.

9. Use according to claim 8, characterized in that the aliphatic thio-compound with an -SAu group is the compound of the formula :
CH₃-CH₂-CH₂-S-Au Na₂So₃

10. Use according to one of claims 1 to 9, characterized in that the thio-glucose is in combination with Zidovudine (AZT) or a derivative thereof, particularly a phosphorylated derivative.

11. Medicinal composition characterized in that it comprises as active principle an effective amount of a pharmacologically or veterinary acceptable aliphatic organic thio-compound having at least an S-Au bond with exclusion of aurothiomalate and aurothiopropanolsulfonate.

12. Composition according to claim 11, characterized in that the aliphatic thio-compound with an -SAu group is the compound of the formula :
CH₃-CH₂-CH₂-S-Au Na₂So₃

13. Medicinal composition according to one of claims 11 and 12, characterized in that it is provided for the treatment of diseases caused by HIV-1, HIV-2, HTLV-1, HTLV-2, AMV, ELAV and FLV virus.

14. Medicinal composition according to one of claims 11 to 13, characterized in that the active principle is encapsulated inside liposomes.

15. Medicinal composition according to one of claims 11 to 14, characterized in that the active principle is in combination with at least an inhibitor of glycosylation, such as 2-desoxy-D-glucose.

16. Medicinal composition according to one of claims 11 to 15, characterized in that the organic thio compound is in combination with Zidovudine (AZT) or a derivative thereof, particularly a phosphorylated derivative.

17. Medicinal composition according to one of claims 11 to 16, characterized in that it is in an intravenously-administrable form.

18. Medicinal composition according to one of claims 11 to 17, characterized in that it is in an orally-administrable form.

## Claims (Claims for the following Contracting State(s): ES)

1. Use of an organic thio-compound having at least an S-Au bond to make a drug useful for the treatment or chimioprophylaxis of diseases caused by retroviruses in the human or animal.

2. Use according to claim 1, characterized in that the compound is a thio-glucose.

3. Use according to claim 2, characterized in that the thio-glucose is a glucose or a glucose analog in which one HO is substituted by an SAu group or an SAuR group in which R represents an organic radical.

4. Use according to one of claims 2 and 3, characterized in that the addition of a C₃-C₈ fatty acyl chain.

5. Use according to claim 4, characterized in that the modified thio-glucose is a thio-glucose modified by octyl-aurothioglucoside.

6. Use according to claim 3, characterized in that the thio-glucose is 1-beta-D-aurothio-glucose.

7. Use according to claim 3, characterized in that the thio-glucose is (2, 3, 4, 6-tetra-o-acetyl-1-thio-beta-D-glucopyranasato-S-triacetylphosphine-gold.

8. Use according to claim 1, characterized in that the thio-compound is aliphatic.

9. Use according to claim 8, characterized in that the aliphatic thio-compound with an -SAu group is the compound of the formula :
CH₃-CH₂-CH₂-S-Au Na₂So₃

10. Use according to one of claims 1 to 9, characterized in that the thio-glucose is in combination with Zidovudine (AZT) or a derivative thereof, particularly a phosphorylated derivative.

11. Method to make a medicinal composition characterized in that one combines as active principle an effective amount of a pharmacologically or veterinary acceptable aliphatic organic thio-compound having at least an-S-Au bond with exclusion of aurothiomalate and aurothiopropanolsulfonate and a suitable vehicule.

12. Method according to claim 11, characterized in that the aliphatic thio-compound with an -SAu group is the compound of the formula :
CH₃-CH₂-CH₂-S-Au Na₂So₃

13. Method according to one of claims 11 and 12, characterized in that the composition is provided for the treatment of diseases caused by HIV-1, HIV-2, HTLV-1, HTLV-2, AMV, ELAV and FLV virus.

14. Method according to one of claims 11 to 13, characterized in that the active principle is encapsulated inside liposomes.

15. Method according to one of claims 11 to 14, characterized in that the active principle is in combination with at least an inhibitor of glycosylation, such as 2-desoxy-D-glucose.

16. Method according to one of claims 11 to 15, characterized in that the organic thio compound is in combination with Zidovudine (AZT) or a derivative thereof, particularly a phosphorylated derivative.

17. Method according to one of claims 11 to 16, characterized in that the composition is in an intravenously-administrable form.

18. Method according to one of claims 11 to 17, characterized in that the composition is in an orally-administrable form.

## Patentansprüche (Patentansprüche für folgende(n) Vertragsstaat(en): AT, BE, CH, DE, DK, FR, GB, IT, LI, LU, NL, SE)

1. Verwendung einer organischen Schwefelverbindung, die wenigstens eine S-Au-Bindung enthält, zur Herstellung eines Medikaments, das für die Behandlung und Chemoprophylaxe von Krankheiten bei Mensch und Tier, die auf Retroviren zurückgehen, bestimmt ist.

2. Verwendung gemäß Anspruch 1, dadurch gekennzeichnet, daß die Verbindung eine Thioglucose ist.

3. Verwendung gemäß Anspruch 2, dadurch gekennzeichnet, daß die Thioglucose eine Glucose oder ein Glucoseanalogon ist, bei der bzw. bei dem eine OH-Gruppe durch eine SAu-Gruppe oder eine SAuR-Gruppe substituiert ist, wobei R einen Rest eines organischen Moleküls darstellt.

4. Verwendung gemäß einem der Ansprüche 2 und 3, dadurch gekennzeichnet, daß die Thioglucose eine Thioglucose ist, die durch die Addition einer C₃₋₈-Fettacylkette modifiziert wurde.

5. Verwendung gemäß Anspruch 4, dadurch gekennzeichnet, daß die modifizierte Thioglucose ein Propyl-, Hexyl-, Octyl-aurothioglucosid ist.

6. Verwendung gemäß Anspruch 3, dadurch gekennzeichnet, daß es sich bei der Thioglucose um 1-β-D-Aurothioglucose handelt.

7. Verwendung gemäß Anspruch 3, dadurch gekennzeichnet, daß es sich bei der Thioglucose um 2,3,4,6-Tetra-O-acetyl-1-thio-β-D-glucopyranosato-S-triacetylphosphingold handelt.

8. Verwendung gemäß Anspruch 1, dadurch gekennzeichnet, daß die Schwefelverbindung aliphatisch ist.

9. Verwendung gemäß Anspruch 8, dadurch gekennzeichnet, daß es sich bei der aliphatischen Schwefelverbindung mit der -SAu-Funktion um die Verbindung mit der Formel
CH₃-CH₂-CH₂-S-Au·Na₂SO₃
handelt.

10. Verwendung gemäß einem der Ansprüche 1 bis 9, dadurch gekennzeichnet, daß die Thioglucose mit Azidovudin (AZT) oder mit einem seiner Derivate, insbesondere einem phosphorylierten Derivat, assoziiert ist.

11. Medikamentöse Zusammensetzung, dadurch gekennzeichnet, daß sie als Wirkstoff eine pharmakologisch oder veterinärmedizinisch wirksame Menge einer organischen aliphatischen Schwefelverbindung, die wenigstens eine S-Au-Bindung enthält, umfaßt, mit Ausnahme von Aurothiomalat und Aurothiopropanolsulfonat.

12. Zusammensetzung gemäß Anspruch 11, dadurch gekennzeichnet, daß es sich bei der aliphatischen Schwefelverbindung mit der -SAu-Funktion um die Verbindung mit der Formel
CH₃-CH₂-CH₂-S-Au·Na₂SO₃
handelt.

13. Medikamentöse Zusammensetzung gemäß einem der Ansprüche 11 und 12, dadurch gekennzeichnet, daß sie zur Behandlung von Krankheiten bestimmt ist, die von den Viren HIV-1, HIV-2, HTLV-1, HTLV-2, AMV, ELAV und FLV hervorgerufen werden.

14. Medikamentöse Zusammensetzung gemäß einem der Ansprüche 11 bis 13, dadurch gekennzeichnet, daß der Wirkstoff im Innern von Liposomen eingeschlossen ist.

15. Medikamentöse Zusammensetzung gemäß einem der Ansprüche 11 bis 14, dadurch gekennzeichnet, daß der Wirkstoff mit wenigstens einem Glycosylierungsinhibitor, wie 2-Desoxy-D-glucose, assoziiert ist.

16. Zusammensetzung gemäß einem der Ansprüche 11 bis 15, dadurch gekennzeichnet, daß die organische Schwefelverbindung mit Azidovudin (AZT) oder mit einem seiner Derivate, insbesondere einem phosphorylierten Derivat, assoziiert ist.

17. Medikamentöse Zusammensetzung gemäß einem der Ansprüche 11 bis 16, dadurch gekennzeichnet, daß sie in einer Form vorliegt, die sich intravenös verabreichen läßt.

18. Medikamentöse Zusammensetzung gemäß einem der Ansprüche 11 bis 17, dadurch gekennzeichnet, daß sie in einer Form vorliegt, die sich oral verabreichen läßt.

## Patentansprüche (Patentansprüche für folgende(n) Vertragsstaat(en): ES)

1. Verwendung einer organischen Schwefelverbindung, die wenigstens eine S-Au-Bindung enthält, zur Herstellung eines Medikaments, das für die Behandlung und Chemoprophylaxe von Krankheiten bei Mensch und Tier, die auf Retroviren zurückgehen, bestimmt ist.

2. Verwendung gemäß Anspruch 1, dadurch gekennzeichnet, daß die Verbindung eine Thioglucose ist.

3. Verwendung gemäß Anspruch 2, dadurch gekennzeichnet, daß die Thioglucose eine Glucose oder ein Glucoseanalogon ist, bei der bzw. bei dem eine OH-Gruppe durch eine SAu-Gruppe oder eine SAuR-Gruppe substituiert ist, wobei R einen Rest eines organischen Moleküls darstellt.

4. Verwendung gemäß einem der Ansprüche 2 und 3, dadurch gekennzeichnet, daß die Thioglucose eine Thioglucose ist, die durch die Addition einer C₃₋₈-Fettacylkette modifiziert wurde.

5. Verwendung gemäß Anspruch 4, dadurch gekennzeichnet, daß die modifizierte Thioglucose ein Propyl-, Hexyl-, Octyl-aurothioglucosid ist.

6. Verwendung gemäß Anspruch 3, dadurch gekennzeichnet, daß es sich bei der Thioglucose um 1-β-D-Aurothioglucose handelt.

7. Verwendung gemäß Anspruch 3, dadurch gekennzeichnet, daß es sich bei der Thioglucose um 2,3,4,6-Tetra-O-acetyl-1-thio-β-D-glucopyranosato-S-triacetylphosphingold handelt.

8. Verwendung gemäß Anspruch 1, dadurch gekennzeichnet, daß die Schwefelverbindung aliphatisch ist.

9. Verwendung gemäß Anspruch 8, dadurch gekennzeichnet, daß es sich bei der aliphatischen Schwefelverbindung mit der -SAu-Funktion um die Verbindung mit der Formel
CH₃-CH₂-CH₂-S-Au·Na₂SO₃
handelt.

10. Verwendung gemäß einem der Ansprüche 1 bis 9, dadurch gekennzeichnet, daß die Thioglucose mit Azidovudin (AZT) oder mit einem seiner Derivate, insbesondere einem phosphorylierten Derivat, assoziiert ist.

11. Verfahren zur Herstellung einer medikamentösen Zusammensetzung, dadurch gekennzeichnet, daß man als Wirkstoff eine pharmakologisch oder veterinärmedizinisch wirksame Menge einer organischen aliphatischen Schwefelverbindung, die wenigstens eine S-Au-Bindung enthält, mit Ausnahme von Aurothiomalat und Aurothiopropanolsulfonat, mit einem geeigneten Träger assoziiert.

12. Verfahren gemäß Anspruch 11, dadurch gekennzeichnet, daß es sich bei der aliphatischen Schwefelverbindung mit der -SAu-Funktion um die Verbindung mit der Formel
CH₃-CH₂-CH₂-S-Au·Na₂SO₃
handelt.

13. Verfahren gemäß einem der Ansprüche 11 und 12, dadurch gekennzeichnet, daß die Zusammensetzung zur Behandlung von Krankheiten bestimmt ist, die von den Viren HIV-1, HIV-2, HTLV-1, HTLV-2, AMV, ELAV und FLV hervorgerufen werden.

14. Verfahren gemäß einem der Ansprüche 11 bis 13, dadurch gekennzeichnet, daß der Wirkstoff im Innern von Liposomen eingeschlossen ist.

15. Verfahren gemäß einem der Ansprüche 11 bis 14, dadurch gekennzeichnet, daß der Wirkstoff mit wenigstens einem Glycosylierungsinhibitor, wie 2-Desoxy-D-glucose, assoziiert ist.

16. Verfahren gemäß einem der Ansprüche 11 bis 15, dadurch gekennzeichnet, daß die organische Schwefelverbindung mit Azidovudin (AZT) oder mit einem seiner Derivate, insbesondere einem phosphorylierten Derivat, assoziiert ist.

17. Verfahren gemäß einem der Ansprüche 11 bis 16, dadurch gekennzeichnet, daß die Zusammensetzung in einer Form vorliegt, die sich intravenös verabreichen läßt.

18. Verfahren gemäß einem der Ansprüche 11 bis 17, dadurch gekennzeichnet, daß die Zusammensetzung in einer Form vorliegt, die sich oral verabreichen läßt.
